# EUROPÄISCHE PATENTSCHRIFT

(11) **EP 1 555 991 B1**
(45) Veröffentlichungstag und Bekanntmachung des Hinweises auf die Patenterteilung: **06.12.2006**
(21) Anmeldenummer: 03810394.1
(22) Anmeldetag: 17.09.2003
(51) Int. Cl.: A61Q 5/04

(54) **MITTEL UND VERFAHREN ZUR DAUERHAFTEN HAARVERFORMUNG AUF BASIS VON ORGANISCHEN BORHYDRID-DERIVATEN**
AGENT AND METHOD FOR PERMANENTLY SHAPING HAIR, BASED ON ORGANIC BORON HYDRIDE DERIVATIVES
PROCEDE ET PRODUIT POUR LA DEFORMATION PERMANENTE DES CHEVEUX A BASE DE DERIVES ORGANIQUES D'HYDRURE DE BORE

(30) Priorität: 02.11.2002 DE 10251104
(43) Veröffentlichungstag der Anmeldung: 27.07.2005
(73) Patentinhaber: Wella Aktiengesellschaft, 64274 Darmstadt (DE)
(72) Erfinder: ARLT, Thilo, 1762 Givisiez (CH); CLÉMENT, Pierre,Alain, 1752 Villars-sur-Glâne (CH); BITTERLI, Natalie, 3280 Murten (CH)
(86) Internationale Anmeldenummer: PCT/EP2003/010317
(87) Internationale Veröffentlichungsnummer: WO 2004/041223

(56) Entgegenhaltungen:
- GB-A- 835 247
- US-A- 2 766 760

## Beschreibung

Die vorliegende Erfindung betrifft ein Mittel zur dauerhaften Haarverformung, welches als keratinreduzierenden Wirkstoff bestimmte organische Borhydrid-Derivate enthält sowie ein Verfahren zur dauerhaften Haarverformung unter Verwendung derartiger Mittel.

Bekanntlich beruht die klassische Technik zur Durchführung einer dauerhaften Haarverformung auf zwei Behandlungsschritten: Im ersten Schritt werden die Cystin-Disulfid-Brücken des Haarkeratins durch Einwirken eines Mittels, welches einen reduzierenden Wirkstoff enthält (Verformungsmittel), geöffnet. Sodann wird das Haar in die gewünschte Form gebracht. In einem zweiten Schritt werden die Cystin-Disulfid-Bindungen unter Verwendung eines Fixiermittels, d. h. eines einen oxidierenden Wirkstoff enthaltenden Mittels, wieder geschlossen.

Als klassisches Dauerwellreduktionsmittel wird bisher die Thioglykolsäure, z. B. als Ammonium- oder Monoethanolaminsalz, verwendet. Weitere übliche haarkeratinreduzierende Wirkstoffe sind anorganische Sulfite, 2-Mercapto-propionsäure (Thiomilchsäure), 3-Mercapto-propionsäure, bestimmte Mercaptocarbonsäureester, Cystein und Derivate dieser Verbindungen.

Alle diese Mittel weisen jedoch eine Reihe von Nachteilen auf. Alkalisch eingestellte Zubereitungen auf Basis der Mercaptocarbonsäuren zeigen trotz ausreichender Wirkung eine Haarschädigung, die sich beispielsweise in vermehrt auftretendem Haarbruch äußert. Vielfach belasten diese Mittel auch in unerwünschter Weise die Kopfhaut.

Zudem erfordert der unangenehme Geruch der verwendeten Reduktionsmittel eine intensive Parfümierung der Produkte. Durch Verwendung von 2-Mercaptopropionsäure (Thiomilchsäure) ist man in der Lage, einige der erwähnten Probleme zu lösen. Allerdings weist die Thiomilchsäure im Vergleich zur allgemein verwendeten Thioglykolsäure durch eine schwächere Umformung auf.

Die Mercaptocarbonsäureester, welche eine Haarverformung auch bei niedrigeren pH-Werten ermöglichen, sind bezüglich ihrer Hautverträglichkeit sowie ihres Sensibilisierungsrisikos nicht zufriedenstellend. Anstelle der Mercaptocarbonsäureester wurden auch Mercaptocarbonsäureamide wie Thioglykolsäureamid oder alkyl- bzw. hydroxyalkylsubstituierte Amide verwendet. Diese Stoffe haben, wie die Carbonsäureester, ein hohes Umformungspotential auch bei niedrigen pH-Werten, sind jedoch in Bezug auf die Sensibilisierung noch kritischer als die Ester.

Um einige Nachteile der Thiole zu vermeiden, wurden in der Vergangenheit versucht auch Borhydride als keratinreduzierende Substanz verwenden. Der Einsatz von Alkaliborhydriden für die dauerhafte Umformung von Haaren ist seit 1956 bekannt, z. B. aus US-PS 2766760.

Zur Herabsetzung der Reaktivität der Alkaliborhydride wurden diverse Anstrengungen unternommen. So ist ein Verfahren zur Einbettung der hygroskopischen und entflammbaren Alkaliborhydride in feste und pastenförmige inerte Einbettungsmittel (DE-AS 1069588) oder in einem Träger aus synthetischem Wachs bekannt (GB-PS 835 247). Auch die Einkapselung der Natrium- oder Kaliumborhydride in einem Überzug von Polykondensationsprodukten von Ethylenoxyd, polyoxyethylierten Fettalkoholen oder durch teilweise in Schwefelsäure übergeführten Fettalkoholen, wurde zur Stabilisierung erwogen (DE-AS 1199927).

Ebenso ist ein Verfahren zur dauerhaften Verformung von Haaren mit Natrium- oder Kaliumborhydrid bekannt, indem das Lösungsmittel eine wäßrige, wäßrigalkoholische oder alkoholische Lösung darstellt. (DE-AS 1129657).

Allen Verfahren unter Verwendung von Alkaliborhydriden ist aber gemeinsam, dass die Lösungen kurz vor der Anwendung hergestellt werden müssen, da eine vollständige Stabilisierung der Natrium- und Kaliumborhydride in Wasser oder Alkohol nicht bewerkstelligt werden konnte. Dieser Nachteil des Standes der Technik konnte beseitigt werden, da die in den erfindungsgemäßen Mitteln verwendeten Borhydride in alkoholischer Lösung löslich und stabil sind, also keinen Wasserstoff entwickeln und daher auch nicht entflammbar sind. Die Notwendigkeit, die das Borhydrid enthaltenden Lösungen vor der Anwendung frisch herzustellen entfällt damit.

Gegenstand der vorliegenden Erfindung ist daher ein Mittel zur dauerhaften Verformung der Haare, dadurch gekennzeichnet, dass es als keratinreduzierenden Wirkstoff
(a) ein Tetraalkylammonium-borhydrid der allgemeinen Formel

   (I) N(R₁R₂R₃R₄)⁺ BH₄⁻

   enthält, in der die Reste R₁ bis R₄ unabhängig voneinander geradkettiges oder verzweigtes Alkyl mit 1 bis 10 Kohlenstoffatomen oder Alkylaryl mit 5 bis 6 Ringatomen bedeutet, unter der Voraussetzung, dass nicht alle Reste gleichzeitig Methyl bedeuten oder
(b) ein Boralkoxyhydrid der allgemeinen Formel 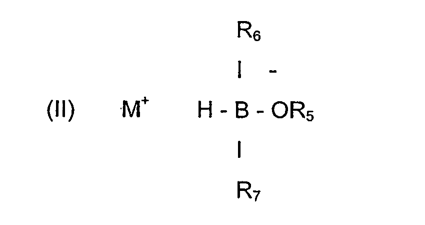 enthält, in dem R₅ die Bedeutung geradkettiges oder verzweigtes Alkyl mit 1 bis 6 Kohlenstoffatomen hat, R₆ und R₇ unabhängig voneinander die Bedeutung H, Halogen, geradkettiges oder verzweigtes Alkyl oder Alkoxy mit 1 bis 6 Kohlenstoffatomen darstellen und M⁺ ein beliebiges anorganisches oder organisches Kation, vorzugsweise Natrium, Kalium oder Ammonium, ist.

Bevorzugte Verbindungen der Formel (I) sind solche, in denen R₁ bis R₄ unabhängig voneinander die Bedeutung -CH₃, -CH₂CH₃, -CH₂CH₂CH₃, -CH₂CH₂CH₂CH₃, -CH(CH₃)₂, -CH₂CH₂CH₂N(CH₃)₂, -CH₂CH₂OCH₃, -CH₂CH₂CH₂OCH₃ oder Benzyl haben.

Besonders bevorzugte Verbindungen der Formel (I) sind solche, in denen mindestens einer der Alkylreste R₁ bis R₄ eine Kettenlänge von mindestens 2 C-Atomen besitzt.

Ganz besonders bevorzugte Verbindungen der Formel (I) sind Tetraethylammoniumborhydrid, Tetrapropylammoniumborhydrid, Tetrabutylammoniumborhydrid und Benzyltriethylammoniumborhydrid.

Bevorzugte Verbindungen der Formel (II) sind jene, in denen alle Substituenten gleich sind und Alkoxy mit 1 bis 6 Kohlenstoffatomen bedeuten.

Besonders bevorzugte Verbindungen der Formel (II) sind Natriumtrimethoxyborhydrid und Kalium-triisopropoxyborhydrid.

Die Borhydride der allgemeinen Formeln (I) oder (II) werden in dem gebrauchsfertigen Mittel zur dauerhaften Haarverformung in einer Menge von insgesamt 1 bis 20 Gewichtsprozent, vorzugsweise 5 bis 15 Gewichtsprozent, eingesetzt.

Die in den erfindungsgemäßen Mitteln enthaltenen Borhydride können in einer weiteren Ausführungsform der Erfindung auch im Gemisch mit mindesten einem bekannten Thiol wie Thioglykolsäure, Thiomilchsäure, Cystein, Cysteamin und Alkyl- oder Acylcysteaminen oder Sulfiten eingesetzt werden.

Das Verformungsmittel kann sowohl ein- als auch zweikomponentig verpackt angeboten werden. Liegt es zweikomponentig vor, dann werden die beiden Komponenten unmittelbar vor dem Gebrauch miteinander vermischt. Das Mittel kann dann sowohl in Form einer wäßrigen, wässrig-alkoholischen, oder alkoholischen Lösung oder einer Emulsion als auch in verdickter Form auf wäßriger Basis, insbesondere als Creme, Gel, Schaum oder Paste vorliegen. Ist es einkomponentig, so liegt es bevorzugt als eine Lösung oder Gel auf der Basis von Alkoholen mit 1 bis 6 Kohlenstoffatomen vor. Die bevorzugten Alkohole sind Ethanol, 1-Propanol, Isopropanol, 1-Butanol, Isobutanol, 1-Pentanol und 1-Hexanol. Auch Lösungen oder Gele in Propylenglycol, Polyglycol, Glycerin oder Tetrahydrofuran sind geeignet.

Selbstverständlich kann das Verformungsmittel alle für derartige Mittel üblichen und bekannten Zusatzstoffe, zum Beispiel Verdickungsmittel, wie beispielsweise Bentonit, Fettsäuren, Stärke, Polyacrylsäure und deren Derivate, Cellulosederivate, Alginate, Vaseline, Paraffinöle; Netzmittel oder Emulgatoren aus den Klassen der anionischen, kationischen, amphoteren oder nichtionogenen oberflächenaktiven Substanzen, beispielsweise Fettalkoholsulfate, Fettalkoholethersulfate, Alkylsulfonate, Alkylbenzolsulfate, quaternäre Ammoniumsalze, Alkylbetaine, oxethylierte Alkylphenole, Fettsäurealkanolamide oder oxethylierte Fettsäureester; ferner Trübungsmittel, wie zum Beispiel Polyethylenglykolester; Alkohole, wie zum Beispiel Ethanol, Propanol, Isopropanol und Glycerin; Zucker wie z. B. D-Glucose; Lösungsvermittler, Stabilisatoren, Puffersubstanzen, Parfümöle, Farbstoffe sowie haarkonditionierende und haarpflegende Bestandteile, wie zum Beispiel kationische Polymere, Lanolinderivate, Cholesterin, Pantothensäure und Betain, enthalten.

Die erwähnten Bestandteile werden in den für solche Zwecke üblichen Mengen verwendet, zum Beispiel die Netzmittel und Emulgatoren in Konzentrationen von insgesamt 0,2 bis 30 Gewichtsprozent, die Alkohole in einer Menge von insgesamt 0,1 bis 20 Gewichtsprozent, die Trübungsmittel, Parfümöle und Farbstoffe in einer Menge von jeweils 0,01 bis 1 Gewichtsprozent, die Puffersubstanzen in einer Menge von insgesamt 0,1 bis 10 Gewichtsprozent, Zucker, Lösungsvermittler, Stabilisatoren, sowie haarkonditionierende und haarpflegende Bestandteile in einer Menge von jeweils 0,1 bis 5 Gewichtsprozent, während die Verdickungsmittel und Lösungsvermittler in einer Menge von insgesamt 0,5 bis 20 Gewichtsprozent in diesem Mittel enthalten sein können.

Weiterhin können diesem Mittel zur Wirkungssteigerung sogenannte Quell- und Penetrationsstoffe, wie zum Beispiel Dipropylenglykolmonomethylether, 2-Pyrrolidon oder lmidazolidin-2-on, in einer Menge von 1 bis 30 Gewichtsprozent sowie zur Vermeidung einer Überkrausung der Haare Dithioverbindungen, beispielsweise Dithiodiglykolsäure, Dithiomilchsäure oder die jeweiligen Salze der Dithiole, zugesetzt werden.

Durch Variation der Konzentration kann ein Mittel zur Verfügung gestellt werden, das zur Anwendung an jeder Haarstruktur, gegebenenfalls unter zusätzlicher Wärmeeinwirkung, geeignet ist. Das Mittel bewirkt eine elastische, dauerhafte und gleichmäßige Umformung vom Haaransatz bis zur Haarspitze.

Die vorliegende Erfindung betrifft weiterhin ein Verfahren zur dauerhaften Haarverformung, bei dem man die Haare bevor und/oder nachdem man sie in die gewünschte Form bringt, mit einem Verformungsmittel behandelt, mit Wasser spült, sodann oxidativ behandelt, mit Wasser spült, gegebenenfalls zur Wasserwelle legt und sodann trocknet, welches dadurch gekennzeichnet ist, daß als Verformungsmittel das vorstehend beschriebene erfindungsgemäße Mittel verwendet wird.

In einer bevorzugten Ausführungsform des erfindungsgemäßen Verfahrens wird das Haar zunächst mit einem Shampoo gewaschen und danach mit Wasser gespült. Anschließend wird das handtuchtrockene Haar in einzelne Strähnen aufgeteilt und auf Wickler mit einem Durchmesser von 5 bis 30 Millimetern, bevorzugt 5 bis 15 Millimeter, gewickelt. Sodann wird das Haar mit einer für die Haarverformung ausreichenden Menge, vorzugsweise 60 bis 120 Gramm, des beschriebenen erfindungsgemäßen Verformungsmittels behandelt.

Nach einer für die dauerhafte Verformung des Haares ausreichenden Einwirkungszeit, welche je nach Haarbeschaffenheit, dem pH-Wert und der Verformungswirksamkeit des Verformungsmittels sowie in Abhängigkeit von der Anwendungstemperatur 5 bis 30 Minuten (10 bis 30 Minuten ohne Wärmeeinwirkung; 5 bis 20 Minuten mit Wärmeeinwirkung) beträgt, wird das Haar mit Wasser gespült und dann oxidativ nachbehandelt ("fixiert"). Das Nachbehandlungsmittel wird, je nach Haarfülle, vorzugsweise in einer Menge von 80 bis 100 Gramm, verwendet.

Für die oxidative Nachbehandlung im aufgewickelten oder abgewickelten Zustand kann jedes beliebige, für eine derartige Behandlung geeignetes Nachbehandlungsmittel verwendet werden. Beispiele für in solchen Nachbehandlungsmitteln verwendbare Oxidationsmittel sind Kalium- und Natriumbromat, Natriumperborat, Harnstoffperoxid und Hydrogenperoxid. Die Konzentration des Oxidationsmittels ist in Abhängigkeit von der Anwendungszeit (in der Regel 5 bis 15 Minuten) und der Anwendungstemperatur unterschiedlich. Normalerweise liegt das Oxidationsmittel in dem gebrauchsfertigen wäßrigen Nachbehandlungsmittel in einer Konzentration von 0,5 bis 10 Gewichtsprozent vor. Das Mittel für die oxidative Nachbehandlung kann selbstverständlich weitere Stoffe, wie zum Beispiel Netzmittel, Pflegestoffe wie kationaktive Polymere, schwache Säuren, Puffersubstanzen oder Peroxidstabilisatoren, enthalten und in Form einer wäßrigen Lösung, einer Emulsion sowie in verdickter Form auf wäßriger Basis, insbesondere als Creme, Gel oder Paste, vorliegen. Diese üblichen Zusätze können insbesondere in einer Menge von 0,1 bis 10 Gew.% in dem Nachbehandlungsmittel enthalten sein.

Anschließend werden die Wickler entfernt. Falls erforderlich, kann das abgewickelte Haar nun nochmals oxidativ nachbehandelt werden. Sodann wird das Haar mit Wasser gespült, gegebenenfalls zur Wasserwelle gelegt und schließlich getrocknet.

Die nachfolgenden Beispiele sollen den Gegenstand der Erfindung näher erläutern.

### Einkomponentige Dauerverformungsmittel

### Beispiel 1: Dauerwellmittel für normales Haar

| | |
|---|---|
| 7,6 g | Tetraethylammonium-borhydrid |
| 0,5 g | ethoxyliertes Polydimethylsiloxan (12EO) (CTFA: PEG-12 DIMETHICONE) |
| 91,9 g | Ethanol |
| 100,0 g | |

Normales, nicht vorgeschädigtes Haar wird gewaschen, mit einem Handtuch frottiert und auf Wickler mit einem Durchmesser von 6 Millimetern gewickelt. Anschließend wird das Haar mit dem vorstehend beschriebenen Haarverformungsmittel gleichmäßig durchfeuchtet. Nach einer Einwirkungszeit von 15 Minuten wird das Haar mit Wasser gründlich gespült und sodann mit 80 Gramm einer 3-prozentigen wäßrigen Hydrogenperoxid-Lösung oxidativ nachbehandelt. Nach Entfernung der Wickler werden die Haare erneut mit Wasser gespült, zur Wasserwelle gelegt und anschließend getrocknet. Das so behandelte Haar besitzt eine gleichmäßige und lebhafte Krause.

### Beispiel 2: Dauerverformungsmittel für normales Haar

| | |
|---|---|
| 12,8 g | Tetrabutylammonium-borhydrid |
| 0,1 g | Parfüm |
| 87,1 g | 1-Butanol |
| 100,0 g | |

Normales, nicht vorgeschädigtes Haar wird gewaschen, mit einem Handtuch frottiert und auf Wickler mit einem Durchmesser von 6 Millimetern gewickelt. Anschließend wird das Haar mit dem vorstehend beschriebenen Haarverformungsmittel gleichmäßig durchfeuchtet. Nach einer Einwirkungszeit von 15 - 25 Minuten wird das Haar mit Wasser gründlich gespült und sodann mit 80 Gramm einer 3-prozentigen wäßrigen Hydrogenperoxid-Lösung oxidativ nachbehandelt. Nach Entfernung der Wickler werden die Haare erneut mit Wasser gespült, zur Wasserwelle gelegt und anschließend getrocknet. Das so behandelte Haar besitzt eine gleichmäßige und lebhafte Krause.

### Beispiel 3: Dauerwellmittel für normales Haar

| | |
|---|---|
| 10,8 g | Benzyltriethylammonium-borhydrid |
| 0,5 g | ethoxylierter Glycerinmonococosfettsäureester (7EO) (CTFA: PEG-7 GLYCERYLCOCOATE) |
| 88,7 g | Isopropanol |
| 100,0 g | |

Normales, nicht vorgeschädigtes Haar wird gewaschen, mit einem Handtuch frottiert und auf Wickler mit einem Durchmesser von 6 Millimetern gewickelt. Anschließend wird das Haar mit dem vorstehend beschriebenen Haarverformungsmittel gleichmäßig durchfeuchtet. Nach einer Einwirkungszeit von 15 - 25 Minuten wird das Haar mit Wasser gründlich gespült und sodann mit 80 Gramm einer 3-prozentigen wässrigen Hydrogenperoxid-Lösung oxidativ nachbehandelt. Nach Entfernung der Wickler werden die Haare erneut mit Wasser gespült, zur Wasserwelle gelegt und anschließend getrocknet. Das so behandelte Haar besitzt eine gleichmäßige und lebhafte Krause.

### Zweikomponentige Dauerverformungsmittel:

### Beispiel 4: Dauerverformungsmittel für normales Haar

| Komponente A | |
|---|---|
| 12,8 g | Tetraethylammonium-borhydrid |

| Komponente B | |
|---|---|
| 1,0 g | Dimethyldiallylammoniumchlorid-homopolymer (CTFA: POLYQUATERNIUM-6) |
| 86,2 g | Wasser |
| 87,2 g | |

Die vorstehenden Komponenten A und B werden unmittelbar vor dem Gebrauch zum gebrauchsfertigen Haarverformungsmittel vermischt. Normales, nicht vorgeschädigtes Haar wird gewaschen, mit einem Handtuch frottiert und auf Wickler mit einem Durchmesser von 6 Millimetern gewickelt. Anschließend wird das Haar mit dem gebrauchsfertigen Haarverformungsmittel gleichmäßig durchfeuchtet. Nach einer Einwirkungszeit von 15 - 25 Minuten wird das Haar mit Wasser gründlich gespült und sodann mit 80 g einer 3-prozentigen wäßrigen Hydrogenperoxidlösung oxidativ nachbehandelt. Nach Entfernung der Wickler werden die Haare erneut mit Wasser gespült, zur Wasserwelle gelegt und anschließend getrocknet. Das so behandelte Haar besitzt eine gleichmäßige und lebhafte Krause.

### Beispiel 5: Dauerverformungsmittel für normales Haar

| Komponente A | |
|---|---|
| 6,4 g | Natrium-trimethoxyborohydrid |

| Komponente B | |
|---|---|
| 1,0 g | Polyethylenglykol (8EO) (CTFA: PEG-8) |
| 92,6 g | 1-Pentanol |
| 93,6 g | |

Die vorstehenden Komponenten A und B werden unmittelbar vor dem Gebrauch zum gebrauchsfertigen Haarverformungsmittel vermischt.. Normales, nicht vorgeschädigtes Haar wird gewaschen, mit einem Handtuch frottiert und auf Wickler mit einem Durchmesser von 6 Millimetern gewickelt. Anschließend wird das Haar mit dem vorstehend beschriebenen Haarverformungsmittel gleichmäßig durchfeuchtet. Nach einer Einwirkungszeit von 15 - 25 Minuten wird das Haar mit Wasser gründlich gespült und sodann mit 80 g einer 3-prozentigen wäßrigen Hydrogenperoxidlösung oxidativ nachbehandelt. Nach Entfernung der Wickler werden die Haare erneut mit Wasser gespült, zur Wasserwelle gelegt und anschließend getrocknet. Das so behandelte Haar besitzt eine gleichmäßige und lebhafte Krause.

## Patentansprüche

1. Mittel zur dauerhaften Verformung der Haare, **dadurch gekennzeichnet, dass** es als keratinreduzierenden Wirkstoff
(a) ein Tetraalkylammonium-borhydrid der allgemeinen Formel
(I) N(R₁R₂R₃R₄)⁺ BH₄⁻
enthält, in der die Reste R₁ bis R₄ unabhängig voneinander geradkettiges oder verzweigtes Alkyl mit 1 bis 10 Kohlenstoffatomen oder Alkylaryl mit 5 bis 6 Ringatomen bedeutet, unter der Voraussetzung, dass nicht alle Reste gleichzeitig Methyl bedeuten oder
(b) ein Boralkoxyhydrid der allgemeinen Formel 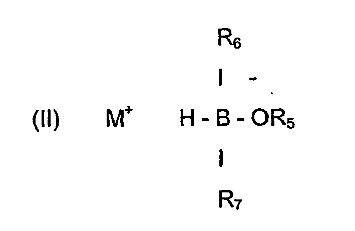 enthält, in dem R₅ die Bedeutung geradkettiges oder verzweigtes Alkyl mit 1 bis 6 Kohlenstoffatomen hat und R₆ und R₇ unabhängig voneinander die Bedeutung H, Halogen, geradkettiges oder verzweigtes Alkyl oder Alkoxy mit 1 bis 6 Kohlenstoffatomen darstellen und M⁺ ein Kation ist.

2. Mittel nach Anspruch 1, **dadurch gekennzeichnet, dass** in der Formel (I) R₁ bis R₄ unabhängig voneinander die Bedeutung -CH₃, -CH₂CH₃, -CH₂CH₂CH₃,- CH₂CH₂CH₂CH₃, -CH(CH₃)₂, -CH₂CH₂CH₂N(CH₃)₂, -CH₂CH₂OCH₃, -CH₂CH₂CH₂OCH₃ oder Benzyl haben.

3. Mittel nach einem der Ansprüche 1 oder 2, **dadurch gekennzeichnet, dass** die Verbindungen der Formel (I) ausgewählt ist aus Tetraethylammonium-borhydrid, Tetrapropylammoniumborhydrid, Tetrabutylammoniumborhydrid, oder Benzyltriethylammoniumborhydrid.

4. Mittel nach Anspruch 1, **dadurch gekennzeichnet, dass** in der Formel (II) alle Substituenten gleich sind und Alkoxy mit 1 bis 6 Kohlenstoffatomen bedeuten.

5. Mittel nach einem der Ansprüche 1 oder 4, **dadurch gekennzeichnet, dass** die Verbindung der Formel (II) ausgewählt ist unter Natrium-trimethoxyborhydrid und Kalium-trüsopropoxyborhydrid.

6. Mittel nach einem der Ansprüche 1 bis 5, **dadurch gekennzeichnet, dass** die Verbindung der Formel (I) und/oder (II) in dem gebrauchsfertigen Mittel in einer Menge von 1 bis 20 Gewichtsprozent enthalten ist.

7. Mittel nach einem der Ansprüche 1 bis 6, **dadurch gekennzeichnet, dass** es zusätzlich mindestens eine haarkeratinreduzierende Verbindung, ausgewählt aus Thioglykolsäure, Thiomilchsäure, Cystein, Cysteamin und Alkyl- oder Acylcysteaminen oder Sulfiten enthält.

8. Mittel nach einem der Ansprüche 1 bis 7, **dadurch gekennzeichnet, dass** es zusätzlich mindestens eine Dithioverbindung enthält.

9. Mittel nach einem der Ansprüche 1 bis 8, **dadurch gekennzeichnet, dass** es als alkoholische Lösung oder Gel vorliegt.

10. Verfahren zur dauerhaften Verformung von Haaren, bei dem man das Haar bevor und/oder nachdem man es in der gewünschten Form festhält, mit einem Verformungsmittel behandelt, mit Wasser spült, oxidativ nachbehandelt, erneut mit Wasser spült, gegebenenfalls zur Wasserwelle legt und sodann trocknet, **dadurch gekennzeichnet, dass** man als Verformungsmittel ein Mittel nach einem der Ansprüche 1 bis 9 verwendet.

## Claims

1. Agent for permanently shaping hair, **characterized in that** it comprises, as keratin-reducing active ingredient,
(a) a tetraalkylammonium borohydride of the general formula
(I) N (R₁R₂R₃R₄)⁺ BH₄⁻
in which the radicals R₁ to R₄, independently of one another, are straight-chain or branched alkyl having 1 to 10 carbon atoms or alkylaryl having 5 to 6 ring atoms, with the proviso that not all of the radicals are methyl at the same time or
(b) an alkoxyborohydride of the general formula 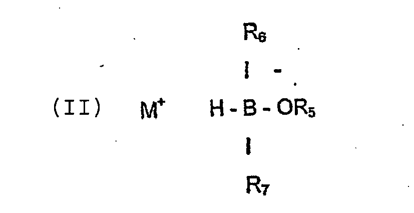 in which R₅ has the meaning straight-chain or branched alkyl having 1 to 6 carbon atoms, and R₆ and R₇, independently of one another, have the meaning H, halogen, straight-chain or branched alkyl or alkoxy having 1 to 6 carbon atoms, and M⁺ is a cation.

2. Agent according to Claim 1, **characterized in that**, in the formula (I), R₁ to R₄, independently of one another, have the meaning -CH₃, -CH₂CH₃, -CH₂CH₂CH₃, -CH₂CH₂CH₂CH₃, -CH(CH₃)₂, -CH₂CH₂CH₂N (CH₃)₂, -CH₂CH₂OCH₃, -CH₂CH₂CH₂OCH₃ or benzyl.

3. Agent according to one of Claims 1 or 2, **characterized in that** the compounds of the formula (I) are chosen from tetraethylammonium borohydride, tetrapropylammonium borohydride, tetrabutylammonium borohydride, or benzyltriethylammonium borohydride.

4. Agent according to Claim 1, **characterized in that**, in the formula (II), all of the substituents are identical and are alkoxy having 1 to 6 carbon atoms.

5. Agent according to one of Claims 1 or 4, **characterized in that** the compound of the formula (II) is chosen from sodium trimethoxyborohydride and potassium triisopropoxyborohydride.

6. Agent according to one of Claims 1 to 5, **characterized in that** the compound of the formula (I) and/or (II) is present in the ready-to-use agent in an amount of from 1 to 20% by weight.

7. Agent according to one of Claims 1 to 6, **characterized in that** it additionally comprises at least one hair-keratin-reducing compound chosen from thioglycolic acid, thiolactic acid, cysteine, cysteamine and alkyl- or acylcysteamines or sulphites.

8. Agent according to one of Claims 1 to 7, **characterized in that** it additionally comprises at least one dithio compound.

9. Agent according to one of Claims 1 to 8, **characterized in that** it is in the form of an alcoholic solution or gel.

10. Method for permanently shaping hair, in which the hair, before and/or after it has been held in the desired shape, is treated with a shaping agent, rinsed with water, oxidatively after-treated, rinsed again with water, optionally set and then dried, **characterized in that** the shaping agent used is an agent according to one of Claims 1 to 9.

## Revendications

1. Composition pour la mise en forme permanente des cheveux, **caractérisée en ce qu'**elle contient en tant que substance active réduisant la kératine
(a) un borohydrure de tétraalkylammonium de formule générale
(I) N(R₁R₂R₃R₄)⁺ BH₄⁻
dans laquelle les radicaux R₁ à R₄ représentent, indépendamment les uns des autres, un groupe alkyle à chaîne droite ou ramifiée ayant de 1 à 10 atomes de carbone ou alkylaryle ayant 5 ou 6 atomes formant le cycle, étant entendu que les radicaux ne représentent pas tous simultanément le groupe méthyle ou
(b) contient un alcoxyhydrure de bore de formule générale 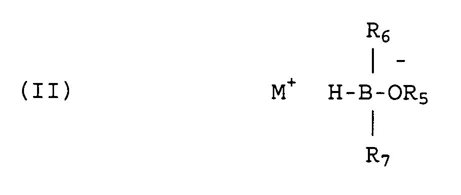 dans laquelle R₅ a la signification d'un groupe alkyle à chaîne droite ou ramifiée ayant de 1 à 6 atomes de carbone et R₆ et R₇ représentent, indépendamment l'un de l'autre, un atome d'hydrogène ou d'halogène, un groupe alkyle ou alcoxy à chaîne droite ou ramifiée ayant de 1 à 6 atomes de carbone, et M⁺ est un cation.

2. Composition selon la revendication 1, **caractérisée en ce que** dans la formule (I) R₁ à R₄ ont indépendamment l'uns de l'autre la signification d'un groupe -CH₃, -CH₂CH₃, -CH₂CH₂CH₃, -CH₂CH₂CH₂CH₃, -CH(CH₃)₂, -CH₂CH₂CH₂N (CH₃)₂, -CH₂CH₂OCH₃, -CH₂CH₂CH₂OCH₃ ou benzyle.

3. Composition selon la revendication 1 ou 2, **caractérisée en ce que** les composés de formule (I) sont choisis parmi le borohydrure de tétraéthylammonium, le borohydrure de tétrapropylammonium, le borohydrure de tétrabutylammonium et le borohydrure de benzyltriéthylamonium.

4. Composition selon la revendication 1, **caractérisée en ce que** dans la formule (II) tous les substituants sont identiques et représentent un groupe alcoxy ayant de 1 à 6 atomes de carbone.

5. Composition selon la revendication 1 ou 4, **caractérisée en ce que** le composé de formule (II) est choisi parmi le triméthoxyborohydrure de sodium et le triisopropoxyborohydrure de potassium.

6. Composition selon l'une quelconque des revendications 1 à 5, **caractérisée en ce que** le composé de formule (I) et/ou de formule (II) est contenu en une quantité de 1 à 20 % en poids dans la composition prête à l'emploi.

7. Composition selon l'une quelconque des revendications 1 à 6, **caractérisée en ce qu'**en outre elle contient au moins un composé réduisant la kératine des cheveux, choisi parmi l'acide thioglycolique, l'acide thiolactique, la cystéine, le cystéamine et des alkyl- ou acylcystéamines ou des sulfites.

8. Composition selon l'une quelconque des revendications 1 à 7, **caractérisée en ce qu'**en outre elle contient au moins un composé dithio.

9. Composition selon l'une quelconque des revendications 1 à 8, **caractérisée en ce qu'**elle se trouve sous forme de solution alcoolique ou de gel.

10. Procédé pour la mise en forme permanente des cheveux, dans lequel les cheveux, avant et/ou après avoir été maintenus en la forme désirée, sont traités par une composition de mise en forme, rincés à l'eau, soumis à un après-traitement par oxydation, de nouveau rincés à l'eau, éventuellement mis en plis et ensuite séchés, **caractérisé en ce qu'**on utilise comme composition de mise en forme une composition selon l'une quelconque des revendications 1 à 9.
